(19) Europäisches Patentamt
European Patent Office
Office européen des brevets

(11) **EP 4 151 201 A1**

(12) **EUROPEAN PATENT APPLICATION**
published in accordance with Art. 153(4) EPC

(43) Date of publication:
**22.03.2023 Bulletin 2023/12**

(21) Application number: **21803810.7**

(22) Date of filing: **14.05.2021**

(51) International Patent Classification (IPC):
*A61K 8/39* (2006.01)     *A61K 8/19* (2006.01)
*A61K 8/25* (2006.01)     *A61K 8/27* (2006.01)
*A61K 8/29* (2006.01)     *A61K 8/36* (2006.01)
*A61K 8/46* (2006.01)     *A61K 8/73* (2006.01)
*A61K 8/81* (2006.01)     *A61Q 1/14* (2006.01)
*A61Q 19/10* (2006.01)

(52) Cooperative Patent Classification (CPC):
**A61K 8/02; A61K 8/19; A61K 8/25; A61K 8/27;
A61K 8/29; A61K 8/36; A61K 8/39; A61K 8/46;
A61K 8/73; A61K 8/81; A61K 8/85; A61Q 1/00;
A61Q 1/14; A61Q 19/10**

(86) International application number:
**PCT/JP2021/018505**

(87) International publication number:
**WO 2021/230374 (18.11.2021 Gazette 2021/46)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.05.2020   JP 2020085342**

(71) Applicant: **Kao Corporation
Chuo-ku
Tokyo 103-8210 (JP)**

(72) Inventors:
• **KAGAYA, Mariko
Tokyo 131-8501 (JP)**
• **HIRAHARA, Mayuko
Tokyo 131-8501 (JP)**
• **TSUDA, Hiroko
Tokyo 131-8501 (JP)**

(74) Representative: **Hoffmann Eitle
Patent- und Rechtsanwälte PartmbB
Arabellastraße 30
81925 München (DE)**

(54) **SHEET PRODUCT**

(57)     Provided is a sheet product containing a sheet, the sheet includes 5 mass% or more of a hydrophobic fiber and is impregnated with a liquid composition including components (A), (B), and (C) below:
(A) a nonionic surfactant: from 0.5 to 9 mass%;
(B) an anionic surfactant having a Krafft point of 45°C or more: from 0.05 to 3 mass% in terms of a salt; and
(C) water: from 70 to 99.45 mass%, and wherein a ratio of a mass of the component (B) in terms of a salt to a mass of the component (A), (B)/(A), is from 0.03 to 1.0.

EP 4 151 201 A1

**Description**

Field of the Invention

The present invention relates to a sheet product.

Background of the Invention

Skin cleansing sheets containing aqueous detergents have been known. For example, Patent Literature 1 describes a skin cleansing sheet composed of specific non-woven fabric impregnated with an aqueous detergent composition containing a nonionic surfactant, polyhydric alcohol and/or glycol ether, and oil content.
Patent Literature 1
JP-A-2001-314342

Summary of the Invention

The present invention relates to a sheet product comprising a sheet, wherein the sheet comprises 5 mass% or more of a hydrophobic fiber and is impregnated with a liquid composition comprising components (A), (B), and (C) below:

(A) a nonionic surfactant: from 0.5 to 9 mass%;
(B) an anionic surfactant having a Krafft point of 45°C or more: from 0.05 to 3 mass% in terms of a salt; and
(C) water: from 70 to 99.45 mass%, and wherein
a ratio of a mass of the component (B) in terms of a salt to a mass of the component (A), (B)/(A), is from 0.03 to 1.0.

Detailed Description of the Invention

The detergency of known skin cleansing sheets mainly depends on the detergency of the aqueous detergent itself. Accordingly, when dirt of the skin (e.g., makeup) is wiped off, the dirt once floated from the skin by the detergency of the aqueous detergent returns to the skin (re-adhesion of dirt). As a result, there is a problem that high detergency cannot be exhibited.
The present invention relates to a sheet product that suppresses re-adhesion of dirt while securing the detergency by the aqueous detergent.
The present inventors found that a sheet product that suppresses re-adhesion of dirt such as makeup, while floating the dirt from the skin, and thereby exhibits high detergency can be obtained by impregnating a sheet with a liquid composition containing a nonionic surfactant and a specific anionic surfactant at a specific ratio.
The sheet product of the present invention can suppress re-adhesion of dirt of the skin such as makeup, while floating the dirt from the skin, and exhibits high detergency. In addition, it is possible to suppress stickiness of the skin and residual feeling after use.
In the present invention, the nonionic surfactant as the component (A) used in the liquid composition may be any nonionic surfactant that is used in ordinary cleansing agents, and examples thereof include sorbitan fatty acid ester such as sorbitan monostearate; polyglycerol fatty acid ester such as glycerol fatty acid ester and polyglyceryl monoisostearate; polyoxyethylene fatty acid ester such as propylene glycol fatty acid ester and polyethylene glycol monolaurate; sucrose fatty acid ester; polyoxyethylene sorbitan fatty acid ester such as polyoxyethylene sorbitan monostearate, polyoxyethylene sorbitan monostearate, and polyoxyethylene coconut oil fatty acid sorbitan; polyoxyethylene alkyl ether; polyoxyethylene sorbitol fatty acid ester; polyoxyethylene glycerol fatty acid ester; polyoxyethylene propylene glycol fatty acid ester; polyoxyethylene castor oil; polyoxyethylene hydrogenated castor oil; polyoxyethylene hydrogenated castor oil fatty acid ester; alkylpolyglucoside; and polyoxyalkylene modified silicone such as a polyoxyethylene-methyl polysiloxane copolymer.
Among these nonionic surfactants, preferred are polyoxyethylene fatty acid ester, polyoxyethylene glycerol fatty acid ester, polyoxyethylene alkyl ether, polyglycerol fatty acid ester, polyoxyethylene sorbitan fatty acid ester, sucrose fatty acid ester, and alkyl polyglucoside, because of high cleansing performance against greasy dirt and water-soluble dirt.
As the component (A), nonionic surfactants can be used singly or in combination of two or more thereof, and the content of the component (A) in the liquid composition is, from the point of making it easier to float dirt from the skin, 0.5 mass% or more, preferably 1.5 mass% or more, and more preferably 2.5 mass% or more and is, from the point of suppressing re-adhesion of dirt and suppressing stickiness of the skin after wiping, 9 mass% or less, preferably 7.5 mass% or less, and more preferably 5 mass% or less. The content of the component (A) in the liquid composition is from 0.5 to 9 mass%, preferably from 1.5 to 7.5 mass%, and more preferably from 2.5 to 5 mass%.

The anionic surfactant as the component (B) used in the liquid composition has a Krafft point of 45°C or more.

While the nonionic surfactant as the component (A) affects the dirt and floats it from the skin, the concentration of the component (A) in the liquid composition decreases. On this occasion, the anionic surfactant as the component (B) dissolved or finely dispersed in the liquid composition has a Krafft point of 45°C or more and a low water solubility and therefore precipitates on the surface of the floated dirt. Consequently, aggregation of the floated dirt progresses to suppress dispersion of the dirt in the liquid composition. As a result, the dirt is likely to be adsorbed to or caught in the sheet and is therefore unlikely to be dispersed in the liquid composition to suppress the return of the dirt to the skin (i.e., a state of the dirt being dispersed in the liquid again and returning and re-adhering to the skin). In addition, since the dirt is unlikely to move to the rear side of the sheet, the offset of the dirt is unlikely to occur. The effects above are derived from the composition of the liquid composition and can be obtained regardless of the structure and composition of the sheet. On this occasion, if the sheet includes a hydrophobic fiber, adsorption of the dirt is accelerated, and the effects are enhanced.

The present invention uses a view that the Krafft point is a melting point of a hydrated solid of a surfactant. That is, the Krafft point is the temperature at which a surfactant precipitates when an aqueous solution thereof is cooled. In some kinds of surfactants, the Krafft point changes depending on the concentration. In the present invention, an anionic surfactant having a Krafft point of 45°C or more at a concentration of 10 mass% in an aqueous solution is used. The Krafft point can be measured using a differential scanning calorimetry.

The anionic surfactant is not particularly limited as long as it has a Krafft point of 45°C or more at a concentration of 10 mass% in an aqueous solution and is used in ordinary skin cleansing agents or cosmetics, and examples thereof include polyoxyalkylene alkyl (or alkenyl) ether sulfates, alkyl (or alkenyl) sulfates, alkyl sulfonates, ether carboxylates, amide ether carboxylates, higher fatty acid salts, alkyl phosphate ester salts, N-acyl amino acid salts, acylated isethionates, acylated taurates, N-alkylamide alkanol sulfate ester salts, and polyoxyalkylene fatty acid amide ether sulfates. Among these anionic surfactants, from the point of smoothness of the skin after wiping, preferred are higher fatty acid salts (e.g., sodium salts having 14 or more carbon atoms and potassium salts having 18 or more carbon atoms), N-acyl amino acid salts (e.g., sodium or potassium salts having 12 or more carbon atoms), and more preferred are sodium stearate, sodium N-stearoyl-N-methyl taurate, and monosodium N-stearoyl glutamate.

As the component (B), anionic surfactants can be used singly or in combination of two or more thereof. The content of the component (B) in terms of a salt in the liquid composition is, from the point of suppressing re-adhesion of dirt, 0.05 mass% or more, preferably 0.1 mass% or more, and more preferably 0.2 mass% or more and is, from the point of suppressing residual feeling on the skin after wiping, 3 mass% or less, preferably 2.7 mass% or less, more preferably 1.5 mass% or less, and further preferably 1 mass% or less. The content of the component (B) in terms of a salt in the liquid composition is from 0.05 to 3 mass%, preferably from 0.1 to 2.7 mass%, more preferably from 0.2 to 1.5 mass%, and further preferably from 0.2 to 1 mass%.

In the liquid composition, the ratio of the mass of the component (B) in terms of a salt to the mass of the component (A), (B)/(A), is, from the point of suppressing re-adhesion of dirt, 0.03 or more, preferably 0.04 or more, and more preferably 0.06 or more and is, from the point of suppressing residual feeling on the skin after wiping, 1.0 or less, preferably 0.6 or less, more preferably 0.45 or less, and further preferably 0.3 or less. The ratio of the mass of the component (B) in terms of a salt to the mass of the component (A), (B)/(A), is from 0.03 to 1.0, preferably from 0.04 to 0.6, more preferably from 0.06 to 0.45, and further preferably from 0.06 to 0.3.

In the liquid composition, water as the component (C) functions as a solvent for dissolving other components.

The content of the component (C) in the liquid composition is, from the point of improving removal of dirt and increasing clean feeling, 70 mass% or more, preferably 75 mass% or more, and more preferably 80 mass% or more and 99.45 mass% or less, preferably 98 mass% or less, and further preferably 97 mass% or less. The content of the component (C) in the liquid composition is from 70 to 99.45 mass%, further preferably from 75 to 98 mass%, and more preferably from 80 to 97 mass%.

The liquid composition can further contain a water-soluble polymer as a component (D). The water-soluble polymer as the component (D) is concentrated on the surface of the sheet to improve the sticking property of the sheet product to the skin and increase the adhesion between the skin and the liquid composition. As a result, the ability of removing dirt on the skin (makeup removability) can be improved even if the amount of the nonionic surfactant as the component (A) in the liquid composition is the same. In addition, the viscosity of the liquid composition is increased to reduce the friction of the skin by the sheet product and improve the feeling of touching the skin. Furthermore, the transpiration rate of water is adjusted to maintain the moisturizing feeling after wiping.

Examples of the water-soluble polymer as the component (D) include an acrylic acid-alkyl (meth)acrylate copolymer, such as an acrylic acid/alkyl acrylate crosspolymer, and cellulose to which a hydroxyalkyl group is added.

As the acrylic acid-alkyl (meth)acrylate copolymer, for example, commercial products, such as Pemulen TR-1, Pemulen TR-2, Carbopol ETD2020, Carbopol 1382, and Carbopol 1342 (they are manufactured by Lubrizol Advanced Materials Inc.) and AQUPEC HV-501ER (manufactured by Sumitomo Seika Chemicals Co., Ltd.), can be used.

Examples of the cellulose to which a hydroxyalkyl group is added include hydroxyethyl cellulose, hydroxypropyl cellulose, hydroxyethyl methyl cellulose, and hydroxypropyl methyl cellulose. For example, as the hydroxypropyl cellulose, commercial products, such as NISSO HPC (manufactured by Nippon Soda Co., Ltd.), can be used.

The water-soluble polymer as the component (D) preferably includes an alkyl group having 3 or more carbon atoms, such as a propyl group, an isopropyl group, or a butyl group, from the point of suppressing re-adhesion of dirt.

As the component (D), water-soluble polymers can be used singly or in combination of two or more thereof. The content of the component (D) in the liquid composition is, from the point of improving the detergency against the skin and the feeling of touching the skin and maintaining the moisturizing feeling after wiping, preferably 0.01 mass% or more, more preferably 0.03 mass% or more, and further preferably 0.05 mass% or more and is, from the point of suppressing the stickiness of the skin after wiping, preferably 0.3 mass% or less, further preferably 0.25 mass% or less, and further preferably 0.2 mass% or less. The content of the component (D) in the liquid composition is preferably from 0.01 to 0.3 mass%, more preferably from 0.03 to 0.25 mass%, and more preferably from 0.05 to 0.2 mass%.

The viscosity of the liquid composition containing the component (D) at 30°C is, from the point of improving the detergency against the skin and the feeling of touching the skin, preferably 5 mPa·s or more and is, from the point of improving the impregnation of the liquid composition into the sheet, preferably 150 mPa·s or less.

In the present invention, the viscosity at 30°C is measured with a digital viscometer (TVB-10, manufactured by Toki Sangyo Co., Ltd., rotor No. 2, 60 rpm) under conditions of 30°C.

The liquid composition can further contain a powder as a component (E). The sticky feeling after wiping is further reduced and smooth feeling of the skin can be provided by containing the component (E).

As the powder as the component (E), any powder that is used in ordinary cosmetics can be used.

As the powder, examples of inorganic powder include talc, silicic anhydride (silica), mica, kaolin, sericite, titanium oxide-coated mica (mica titanium), zinc oxide, titanium oxide, magnesium sulfate, and magnesium carbonate.

Among these powders, from the point of reducing the sticky feeling after wiping and improving the smooth feeling of the skin, it is preferable to include one or more selected from the group consisting of talc, titanium oxide, silicic anhydride (silica), mica, and kaolin, more preferably to include one or more selected from the group consisting of talc, titanium oxide, and silicic anhydride (silica), and further preferably to include one or more selected from the group consisting of talc and silicic anhydride (silica).

Examples of organic powder include polymethyl methacrylate, polymethyl silsesquioxane, a dimethylpolysiloxane polymer, a (lauryl methacrylate/sodium methacrylate) crosspolymer, and aluminum starch octenylsuccinate.

Among these powders, from the point of reducing sticky feeling after wiping and improving the smooth feeling of the skin, it is preferable to include one or more selected from the group consisting of polymethyl silsesquioxane, a dimethylpolysiloxane polymer, and a (lauryl methacrylate/sodium methacrylate) crosspolymer and more preferably to include a (lauryl methacrylate/sodium methacrylate) crosspolymer.

As the component (E), powders can be used singly or in combination of two or more thereof. The content of the component (E) in the liquid composition is, from the point of reducing the sticky feeling after wiping and providing smooth feeling of the skin, preferably 0.05 mass% or more, more preferably 0.07 mass% or more, and further preferably 0.1 mass% or more and, from the point of suppressing the residual feeling to the skin, preferably 10 mass% or less, more preferably 9 mass% or less, and further preferably 7 mass% or less. The content of the component (E) in the liquid composition is preferably from 0.05 to 10 mass%, more preferably from 0.07 to 9 mass%, and further preferably from 0.1 to 7 mass%.

The liquid composition can contain, in addition to the above components, components that are used in ordinary skin cleansing agents, for example, a surfactant other than the above-mentioned components, an oily component, a moisturizer, a bactericide, alcohols, an anti-inflammatory agent, a preservative, a chelating agent, salts, a pearling agent, an aromatic agent, a cooling sensation agent, a dye, a UV absorber, an antioxidant, and a plant extract.

Examples of the oily component include ester oil, silicone oil, and hydrocarbon oil. When an oily component is contained, the content of the oily component (excluding the component (A)) in the liquid composition is, from the viewpoint of suppressing the re-adhesion of dirt, preferably 18 mass% or less, more preferably 15 mass% or less, and further preferably 10 mass% or less.

The liquid composition can be manufactured by a general method. For example, the liquid composition can be manufactured by weighing each of the components and mixing and stirring them. On this occasion, heating may be performed as needed. In particular, it is preferable to sufficiently dissolve them by heating to a temperature of the Krafft point or more of the component (B).

In the present invention, such a liquid composition is used by being impregnated in a sheet.

The sheet used in the present invention is not particularly limited, and any cloth-like sheet including a natural or synthetic fiber, for example, a woven fabric, a knitted fabric, felt, or a non-woven fabric, can be used.

Examples of the natural fiber include a cellulose fiber.

The sheet to be impregnated with the liquid composition is preferably a non-woven fabric from the viewpoint of the followability to three-dimensional shape, improvement in feeling of use, ease in processing, and so on.

The method for manufacturing the non-woven fabric may be, for example, heat fusion, span bonding, or water flow entanglement (span lace).

The sheet preferably includes a hydrophobic fiber from the point of easily suppressing the re-adhesion of dirt and capable of releasing a large amount of the impregnation liquid when wiping.

Examples of the hydrophobic fiber include synthetic fibers, for example, a polyolefin fiber, such as polyethylene and polypropylene; a polyester fiber, such as polyethylene terephthalate; a polyamide fiber, such as Nylon; a polyacrylic nitrile fiber; a polyvinyl alcohol fiber; and a polyurethane fiber.

Among these fibers, one or more selected from the group consisting of polyolefin fibers and polyester fibers are preferably included because of the low prices and good availability in the market. In addition, a fiber obtained by heat fusion of a plurality of materials can also be used, or a composite fiber, such as a coresheath type or a parallel type, can also be used.

The sheet preferably includes a hydrophobic fiber preferably in an amount of 5 mass% or more, more preferably 10 mass% or more, and further preferably 15 mass% or more, in the point of more easily suppressing the re-adhesion of dirt and releasing a large amount of the impregnation liquid when wiping.

In addition, the sheet preferably includes a hydrophilic fiber from the point of reducing the friction feeling of the skin and improving the feeling of touching the skin.

Examples of the hydrophilic fiber include cotton, rayon, and pulp.

Among these fibers, one or more selected from the group consisting of cotton and rayon are preferably included because of the low prices and good availability in the market and from the point of improving the adhesion to the skin and the good feeling of touching the skin. When cotton and rayon are used in combination, any mixing proportion may be adopted in the above-mentioned point.

The sheet includes a hydrophilic fiber preferably in an amount of 10 mass% or more, more preferably 30 mass% or more, and further preferably 65 mass% or more from the point of further reducing the friction feeling of the skin and further improving the feeling of touching the skin.

These hydrophobic fibers and hydrophilic fibers may be used, respectively, singly or in combination of two or more.

When the sheet includes a hydrophobic fiber and a hydrophilic fiber, the mass ratio of the hydrophobic fiber and the hydrophilic fiber, hydrophobic fiber/hydrophilic fiber, is preferably from 5/95 to 70/30 and more preferably from 15/85 to 35/65, from the point of simultaneously achieving suppression of the re-adhesion of dirt, release of a large amount of the impregnation liquid when wiping, reduction in the friction feeling of the skin, and improvement in the feeling of touching the skin.

The density of the sheet is, from the point of allowing a large amount of the impregnation liquid to be released when wiping and reducing the friction of the skin by the sheet product, preferably 0.14 $g/cm^3$ or less, more preferably 0.12 $g/cm^3$ or less, and further preferably 0.11 $g/cm^3$ or less. In addition, the density of the sheet is preferably 0.08 $g/cm^3$ or more and more preferably 0.1 $g/cm^3$ or more from the point of allowing the sheet to be easily impregnated with the liquid composition and improving the retention of the composition on the sheet.

The basis weight of the sheet is preferably from 35 to 80 $g/m^2$, more preferably from 38 to 60 $g/m^2$, and further preferably from 40 to 50 $g/m^2$ from the point of allowing a large amount of the impregnation liquid to be released when wiping and reducing the friction of the skin by the sheet product.

The thickness of the sheet is preferably from 0.3 to 0.6 mm, more preferably from 0.35 to 0.55 mm, and further preferably from 0.38 to 0.5 mm from the point of ease in wiping off and maintaining good feeling of touching the skin.

The shape and size of the sheet are not particularly limited and can be appropriately set according to the purpose.

The sheet may be a multilayer sheet in which two or more sheets made of one kind or two or more kinds (different in, for example, composition) are laminated or may be a laminate of woven fabric, a laminate of non-woven fabric, a laminate of woven fabric and non-woven fabric, or the like. In such a case, as the basis weight and thickness, those of the whole multilayer sheet can be used.

The sheet product of the present invention is made by impregnating such a sheet with the liquid composition.

The rate of impregnation of the liquid composition to the sheet is preferably from 250 to 700 mass%, more preferably from 300 to 600 mass%, based on the sheet mass from the point of good feeling when wiping.

The rate of impregnation can be set as an increment when a sheet is impregnated with a certain amount of a liquid composition with respect to the sheet before the impregnation with the liquid composition.

The method for impregnating a sheet with a liquid composition is not particularly limited, and examples thereof include a method in which a liquid composition is dropwise added or sprayed to a sheet and a method in which a sheet is immersed in a liquid composition. After impregnation with a liquid composition, in order to prevent volatilization of components from the composition, it is preferable to pack the sheet in, for example, an aluminum pouch having high sealing performance.

The sheet impregnated with a liquid composition is suitable as a skin cleansing sheet and can be used for cleansing the dirt of the skin, for example, makeup cosmetics such as foundation, blusher, and eye shadow and dirt such as solid fat among sebum. In particular, since an effect of suppressing re-adhesion of dirt is noticeable, it is suitably used for removing makeup cosmetics.

The skin cleansing sheet of the present invention can be used by, for example, pressing the sheet against the part where removal of dirt such as makeup cosmetics is desired and wiping off the dirt.

A skin cleansing sheet (sheet-shaped makeup remover) for removing makeup cosmetics (hereinafter, simply referred to as makeup) is characterized by its convenience of allowing makeup to be removed anytime anywhere. On the other hand, there are problems of "makeup removability" and "skin friction". One of the big reasons thereof is re-adhesion of dirt (return to the skin), which caused by the makeup wiped off once is dispersed in the impregnation liquid and returns to the skin. The dirt does not come off due to the return to the skin. Furthermore, wiping is performed many times, which leads to skin friction. The present invention has realized a sheet-shaped makeup remover that can suppress the return of dirt to the skin and remove makeup without rubbing many times based on a new idea of adsorbing dirt on the sheet. This is technology that leads to the solution of both of the above-mentioned problems of a sheet-shaped makeup remover: "makeup removability" and "skin friction".

Regarding the above-described embodiments, the present invention further discloses the following sheet products and so on.

<1> A sheet product comprising a sheet, wherein the sheet comprises 5 mass% or more of a hydrophobic fiber and is impregnated with a liquid composition comprising components (A), (B), and (C) below:

(A) a nonionic surfactant: from 0.5 to 9 mass%;
(B) an anionic surfactant having a Krafft point of 45°C or more: from 0.05 to 3 mass% in terms of a salt; and
(C) water: from 70 to 99.45 mass%, and wherein a ratio of a mass of the component (B) in terms of a salt to a mass of the component (A), (B)/(A), is from 0.03 to 1.0.

<2> The sheet product according to the above <1>, wherein the component (A) is preferably polyoxyethylene fatty acid ester, polyoxyethylene glycerol fatty acid ester, polyoxyethylene alkyl ether, polyglycerol fatty acid ester, polyoxyethylene sorbitan fatty acid ester, sucrose fatty acid ester, or alkyl polyglucoside and more preferably polyoxyethylene fatty acid ester or polyoxyethylene glycerol fatty acid ester.

<3> The sheet product according to the above <1> or <2>, wherein a content of the component (A) in the liquid composition is preferably 1.5 mass% or more and more preferably 2.5 mass% or more and preferably 7.5 mass% or less and more preferably 5 mass% or less.

<4> The sheet according to any one of the above <1> to <3>, wherein the component (B) is preferably a higher fatty acid salt or an N-acyl amino acid salt and more preferably sodium stearate, sodium N-stearoyl-N-methyl taurate, or monosodium N-stearoyl glutamate.

<5> The sheet product according to any one of the above <1> to <4>, wherein a content of the component (B) in the liquid composition is preferably 0.1 mass% or more and more preferably 0.2 mass% or more and preferably 2.7 mass% or less, more preferably 1.5 mass% or less, and further preferably 1 mass% or less.

<6> The sheet product according to any one of the above <1> to <5>, wherein the ratio of the mass of the component (B) in terms of a salt to the mass of the component (A), (B)/(A), in the liquid composition is preferably 0.04 or more and more preferably 0.06 or more and preferably 0.6 or less, more preferably 0.45 or less, and further preferably 0.3 or less.

<7> The sheet product according to any one of the above <1> to <6>, wherein a content of the component (C) in the liquid composition is preferably 75 mass% or more and more preferably 80 mass% or more and preferably 98 mass% or less and more preferably 97 mass% or less.

<8> The sheet product according to any one of the above <1> to <7>, wherein the liquid composition further comprises (D) a water-soluble polymer.

<9> The sheet product according to the above <8>, wherein the component (D) is preferably an acrylic acid-alkyl (meth)acrylate copolymer or cellulose to which a hydroxyalkyl group is added and more preferably an acrylic acid-alkyl (meth)acrylate copolymer or hydroxypropyl cellulose.

<10> The sheet product according to the above <8> or <9>, wherein the component (D) preferably includes an alkyl group having 3 or more carbon atoms.

<11> The sheet product according to any one of the above <8> to <10>, wherein a content of the component (D) in the liquid composition is preferably 0.01 mass% or more, more preferably 0.03 mass% or more, and further preferably 0.05 mass% or more and preferably 0.3 mass% or less, more preferably 0.25 mass% or less, and further preferably 0.2 mass% or less.

<12> The sheet product according to any one of the above <8> to <11>, wherein the viscosity of the liquid composition containing the component (D) at 30°C is preferably 5 mPa·s or more and preferably 150 mPa·s or less.

<13> The sheet product according to any one of the above <1> to <12>, wherein the liquid composition further comprises (E) a powder.

<14> The sheet product according to the above <13>, wherein the component (E) is preferably an inorganic powder, more preferably includes one or more selected from the group consisting of talc, titanium oxide, silicic anhydride (silica), mica, and kaolin, further preferably includes one or more selected from the group consisting of talc, titanium oxide, and silicic anhydride (silica), and further more preferably includes one or more selected from the group consisting of talc and silicic anhydride (silica).

<15> The sheet product according to the above <13>, wherein the component (E) is preferably an organic powder, more preferably includes one or more selected from the group consisting of polymethyl silsesquioxane, a dimethylpolysiloxane polymer, and a (lauryl methacrylate/sodium methacrylate) crosspolymer, and further preferably includes a (lauryl methacrylate/sodium methacrylate) crosspolymer.

<16> The sheet product according to any one of the above <13> to <15>, wherein a content of the component (E) in the liquid composition is preferably 0.05 mass% or more, more preferably 0.07 mass% or more, and further preferably 0.1 mass% or more and preferably 10 mass% or less, more preferably 9 mass% or less, and further preferably 7 mass% or less.

<17> The sheet product according to any one of the above <1> to <16>, wherein the liquid composition further comprises an oily component other than the component (A).

<18> The sheet product according to the above <17>, wherein a content of the oily component other than the component (A) in the liquid composition is preferably 18 mass% or less, more preferably 15 mass% or less, and further preferably 10 mass% or less.

<19> The sheet product according to any one of the above <1> to <18>, wherein the hydrophobic fiber is preferably a synthetic fiber selected from the group consisting of a polyolefin fiber, a polyester fiber, a polyamide fiber, a polyacrylic nitrile fiber, a polyvinyl alcohol fiber, and a polyurethane fiber and more preferably includes one or more selected from the group consisting of a polyolefin fiber and a polyester fiber.

<20> The sheet product according to any one of the above <1> to <19>, wherein the sheet includes the hydrophobic fiber preferably in an amount of 10 mass% or more, more preferably 15 mass% or more.

<21> The sheet product according to any one of the above <1> to <20>, wherein the sheet preferably contains a hydrophilic fiber.

<22> The sheet product according to the above <21>, wherein the hydrophilic fiber preferably includes one or more selected from the group consisting of cotton and rayon.

<23> The sheet product according to the above <21> or <22>, wherein the sheet includes the hydrophilic fiber preferably in an amount of 10 mass% or more, more preferably 30 mass% or more, and further preferably 65 mass% or more.

<24> The sheet product according to any one of the above <1> to <23>, wherein the sheet preferably includes a hydrophobic fiber and a hydrophilic fiber, and a mass ratio of the hydrophobic fiber to the hydrophilic fiber, hydrophobic fiber/hydrophilic fiber, is preferably from 5/95 to 70/30, more preferably from 15/85 to 35/65.

<25> The sheet product according to any one of the above <1> to <24>, wherein the sheet preferably has a density of 0.14 g/cm$^3$ or less, more preferably 0.12 g/cm$^3$ or less, and further preferably 0.11 g/cm$^3$ or less and preferably 0.08 g/cm$^3$ or more and more preferably 0.1 g/cm$^3$ or more.

<26> The sheet product according to any one of the above <1> to <25>, wherein the sheet preferably has a basis weight of from 35 to 80 g/m$^2$, more preferably from 38 to 60 g/m$^2$, and further preferably from 40 to 50 g/m$^2$.

<27> The sheet product according to any one of the above <1> to <26>, wherein the sheet preferably has a thickness of from 0.3 to 0.6 mm, more preferably from 0.35 to 0.55 mm, and further preferably from 0.38 to 0.5 mm.

<28> The sheet product according to any one of the above <1> to <27>, wherein the rate of impregnation of the liquid composition to the sheet is preferably from 250 to 700 mass% and more preferably from 300 to 600 mass% based on the sheet mass.

<29> The sheet product according to any one of the above <1> to <28>, wherein the sheet product is for skin cleansing.

<30> The sheet product according to any one of the above <1> to <29>, wherein the sheet product is for removing makeup cosmetics.

<31> The sheet product according to any one of the above <1> to <30>, wherein the sheet product is to be used by pressing the sheet product against a part where removal of dirt is desired and wiping off the dirt.

<32> A skin cleansing method comprising:

applying a sheet product to the skin for wiping, wherein the sheet product comprises a sheet, wherein the sheet comprises 5 mass% or more of a hydrophobic fiber and is

impregnated with a liquid composition comprising components (A), (B), and (C) below:

(A) a nonionic surfactant: from 0.5 to 9 mass%;
(B) an anionic surfactant having a Krafft point of 45°C or more: from 0.05 to 3 mass% in terms of a salt; and

(C) water: from 70 to 99.45 mass%, and wherein a ratio of a mass of the component (B) in terms of a salt to a mass of the component (A), (B)/(A), is from 0.03 to 1.0.

<33> A skin cleansing method comprising:

impregnating a sheet comprising 5 mass% or more of a hydrophobic fiber with a liquid composition comprising components (A), (B), and (C) below:

(A) a nonionic surfactant: from 0.5 to 9 mass%;
(B) an anionic surfactant having a Krafft point of 45°C or more: from 0.05 to 3 mass% in terms of a salt; and
(C) water: from 70 to 99.45 mass%, wherein a ratio of a mass of the component (B) in terms of a salt

to a mass of the component (A), (B)/(A), is from 0.03 to 1.0; and
applying the sheet to the skin for wiping.

<34> A skin cleansing method comprising:
applying the sheet product according to any one of the above <1> to <31> to makeup cosmetics on the skin for wiping.
<35> Use of the sheet product according to any one of the above <1> to <31> as a skin cleansing sheet.
<36> Use of the sheet product according to any one of the above <1> to <31> as a sheet for removing makeup cosmetics.
<37> A method for manufacturing a skin cleansing sheet, comprising:

producing a liquid composition by blending components (A), (B), and (C) below:

(A) a nonionic surfactant: from 0.5 to 9 mass%;
(B) an anionic surfactant having a Krafft point of 45°C or more: from 0.05 to 3 mass% in terms of a salt; and
(C) water: from 70 to 99.45 mass%,

such that a ratio of a mass of the component (B) in terms of a salt to a mass of the component (A), (B)/(A), is from 0.03 to 1.0; and
impregnating the liquid composition to a sheet including 5 mass% or more of a hydrophobic fiber.

Example

Examples 1 to 23 and Comparative Examples 1 to 5

Liquid compositions having compositions shown in Tables 1 to 4 were prepared and impregnated to a non-woven fabric sheet shown in Table 5 to manufacture skin cleansing sheets.
The resulting skin cleansing sheets were evaluated for the dirt (makeup) removal, re-adhesion suppressing effect, and usability (feeling of touching the skin and the skin after use (no stickiness and no residual feeling)). The results are collectively shown in Tables 1 to 4.
The density of a non-woven fabric sheet was measured by the method shown below.

(Manufacturing method)

(1) Manufacturing of liquid composition:
Each component (mass%) was uniformly mixed, and the mixture was heated to a temperature of the Krafft point or more of the component (B) to prepare a liquid composition.
(2) Manufacturing of skin cleansing sheet:
A non-woven fabric sheet cut into 16 cm × 20 cm in advance was impregnated entirely with a regulated amount of the liquid composition using disposable dropper to obtain a skin cleansing sheet.

(Evaluation method)

(1) Dirt (makeup) removal:

A certain amount of lipstick (Maybelline Color Sensational Lipstick (trademark) RD638, manufactured by NIHON L'OREAL K.K.) was applied to the inside of the human lower arm, and the color difference was measured (EI). The lipstick was then wiped off using each skin cleansing sheet at a constant pressure for a constant number of times (3 times). Subsequently, the color difference was measured again (E2). The cleansing rate was calculated using the measured color differences (E1, E2) by the following equation and was evaluated by the following criteria.

$$\text{Cleansing rate (\%)} = (1 - E2/E1) \times 100$$

The color difference was measured using Minolta color difference meter CR-300 (manufactured by KONICA MINOLTA, Inc.).

    1: cleansing rate: less than 30%,
    2: cleansing rate: 30% or more and less than 40%,
    3: cleansing rate: 40% or more and less than 50%,
    4: cleansing rate: 50% or more and less than 60%, and
    5: cleansing rate: 60% or more.

(2) Re-adhesion suppressing effect:
10 mg of liquid foundation (Sofina Primavista (trademark) Pore/Color Uneven Cover c, manufactured by Kao Corporation) was applied to an artificial skin (Bio Skin (trademark), manufactured by Beaulax Co., Ltd., dry type) having a size of 49 mm × 130 mm and was dried for 1 hour or more. The artificial skin was wiped with a 4-fold skin cleansing sheet 5 times repeatedly, and the wiped sheet surface (wiped surface) was brought into light contact with clean paper waste cloth (Kimtowel (trademark) White, manufactured by NIPPON PAPER CRECIA Co., Ltd.). The foundation adhered to the waste cloth was defined as re-adhesion, and the degree thereof was compared to the foundation adhered to the wiped surface to visually evaluate the re-adhesion by the following criteria. The amount of the foundation adhered to the waste cloth was positively correlated with the color of the liquid squeezed from the skin cleansing sheet after wiping. The evaluation value is indicated by the average value (rounded to the first decimal place) of five specialized panelists.

    1: the foundation adhered to the waste cloth with the same degree as that on the wiped surface,
    2: the foundation adhered to the waste cloth, but the amount thereof was slightly smaller than that on the wiped surface,
    3: the foundation adhered to the waste cloth, but the amount thereof was smaller than that on the wiped surface,
    4: the foundation adhered to the waste cloth, but the amount thereof was significantly smaller than that on the wiped surface, and
    5: the foundation hardly adhered to the waste cloth.

(3) Usability evaluation:
The forearms of five professional female panelists in their 20s to 40s were each wiped in a length of 10 cm with a 4-fold skin cleansing sheet applied thereto at a load of about 300 g five times continuously, and then "feeling of touching the skin", "the skin after use (no stickiness)", and "the skin after use (no residual feeling)" were sensorily evaluated by the following criteria. The evaluation value is indicated by the average value (rounded to the first decimal place) of the five specialized panelists.

(Feeling of touching the skin)

    1: the smoothness on the skin is bad, and hardness is felt in the feeling of touching the skin,
    2: the smoothness on the skin is slightly bad, and slight hardness is felt in the feeling of touching the skin,
    3: the smoothness on the skin is slightly good, and the feeling of touching the skin is also slightly soft,
    4: the smoothness on the skin is good, and the feeling of touching the skin is also soft, and
    5: the smoothness on the skin is very good, and the feeling of touching the skin is also very soft.

(The skin after use (no stickiness))

1: stickiness is felt on the skin strongly,
2: stickiness is felt on the skin a little strongly,
3: stickiness is felt on the skin slightly,
4: almost no stickiness is felt on the skin, and
5: stickiness is not felt on the skin at all, and refreshment is felt.

(The skin after use (no residual feeling))

1: residual feeling on the skin is felt strongly,
2: residual feeling on the skin is felt a little strongly,
3: residual feeling on the skin is felt slightly,
4: almost no residual feeling on the skin is felt, and
5: residual feeling on the skin is not felt at all, and refreshment is felt.

(4) Density of non-woven fabric sheet:

The density of a non-woven fabric sheet was calculated by dividing the basis weight of the non-woven fabric sheet by the thickness of the non-woven fabric sheet.
The thickness of a non-woven fabric sheet was determined as the average of the thicknesses at arbitrary five positions of the non-woven fabric sheet measured using a constant pressured thickness measuring instrument (S-10908 type, manufactured by TECLOCK Co., Ltd.) in accordance with the method A in "Thickness" test of Test methods for nonwovens (JIS L 1913).

[Table 1]

| Component (mass%) | | Example | | | | | | | | | |
|---|---|---|---|---|---|---|---|---|---|---|---|
| | | 1 | 2 | 3 | 4 | 5 | 6 | 7 | 8 | 9 | 10 |
| (A) | Polyoxyethylene (caprylate/ caprate) glycerides (TEGOSOFT GMC 6, manufactured by Evonik Japan Co., Ltd.) | 3 | 3 | 3 | | | 2.5 | 0.5 | 5 | 7.5 | 9 |
| | Polyethylene glycol monolaurate (Emanon 1112HG, manufactured by Kao Corporation) | | | | 3 | 3 | | | | | |

(continued)

| Component (mass%) | | Example | | | | | | | | | |
|---|---|---|---|---|---|---|---|---|---|---|---|
| | | 1 | 2 | 3 | 4 | 5 | 6 | 7 | 8 | 9 | 10 |
| (B) | Sodium N-stearoyl-N-methyltaurate (Kp = 50) (Nikkol SMT, manufactured by Nikko Chemicals Co., Ltd.) | 0.3 | 0.3 | | 0.3 | | 0.25 | 0.05 | 0.5 | 0.75 | 0.9 |
| | Sodium stearate (Kp = 79) | | | 0.3 | | 0.3 | | | | | |
| Solvent | Ethanol | 5 | | 5 | 5 | 5 | 5 | 5 | 5 | 5 | 5 |
| (C) | Water | 91.7 | 96.7 | 91.7 | 91.7 | 91.7 | 92.25 | 94.45 | 89.5 | 86.75 | 85.1 |
| | Total | 100 | 100 | 100 | 100 | 100 | 100 | 100 | 100 | 100 | 100 |
| | (B)/(A) | 0.10 | 0.10 | 0.10 | 0.10 | 0.10 | 0.10 | 0.10 | 0.10 | 0.10 | 0.10 |
| Effect | Dirt (makeup) removal | 4 | 4 | 4 | 4 | 4 | 4 | 3 | 4 | 4 | 4 |
| | Re-adhesion suppressing effect | 5 | 5 | 5 | 5 | 5 | 5 | 5 | 5 | 5 | 3 |
| | Skin after use (no stickiness) | 5 | 5 | 5 | 5 | 5 | 5 | 5 | 5 | 4 | 3 |
| | Skin after use (no residual feeling) | 5 | 5 | 5 | 5 | 5 | 5 | 5 | 5 | 5 | 5 |
| | Feeling of touching the skin | 4 | 4 | 4 | 4 | 4 | 4 | 4 | 4 | 4 | 4 |

[Table 2]

| Component (mass%) | | Example | | | | | | | | |
|---|---|---|---|---|---|---|---|---|---|---|
| | | 11 | 12 | 13 | 14 | 15 | 16 | 17 | 18 | 19 |
| (A) | Polyoxyethylene (caprylate/caprate) glycerides (TEGOSOFT GMC 6, manufactured by Evonik Japan Co., Ltd.) | 5 | 5 | 5 | 3 | 3 | 3 | 3 | 5 | 3 |
| | Polyethylene glycol monolaurate (Emanon 1112HG, manufactured by Kao Corporation) | | | | | | | | | |

(continued)

| Component (mass%) | | Example | | | | | | | | |
|---|---|---|---|---|---|---|---|---|---|---|
| | | 11 | 12 | 13 | 14 | 15 | 16 | 17 | 18 | 19 |
| (B) | Sodium N-stearoyl-N-methyltaurate (Kp = 50) (Nikkol SMT, manufactured by Nikko Chemicals Co., Ltd.) | 1 | 1.5 | 2.7 | 0.18 | 0.14 | 0.09 | 1.35 | 3 | 2 |
| | Sodium stearate (Kp = 79) | | | | | | | | | |
| Solvent | Ethanol | 5 | 5 | 5 | 5 | 5 | 5 | 5 | 5 | 5 |
| (C) | Water | 89 | 88.5 | 87.3 | 91.82 | 91.86 | 91.91 | 90.65 | 87.00 | 90.00 |
| | Total | 100 | 100 | 100 | 100 | 100 | 100 | 100 | 100 | 100 |
| | (B)/(A) | 0.20 | 0.30 | 0.54 | 0.06 | 0.05 | 0.03 | 0.45 | 0.60 | 0.67 |
| Effect | Dirt (makeup) removal | 4 | 4 | 4 | 4 | 4 | 4 | 4 | 4 | 4 |
| | Re-adhesion suppressing effect | 5 | 5 | 5 | 5 | 4 | 3 | 5 | 5 | 5 |
| | Skin after use (no stickiness) | 5 | 5 | 5 | 5 | 5 | 5 | 5 | 5 | 5 |
| | Skin after use (no residual feeling) | 5 | 4 | 3 | 5 | 5 | 5 | 4 | 2 | 2 |
| | Feeling of touching the skin | 4 | 4 | 4 | 4 | 4 | 4 | 4 | 4 | 4 |

[Table 3]

| Component (mass%) | | Comparative Example | | | | |
|---|---|---|---|---|---|---|
| | | 1 | 2 | 3 | 4 | 5 |
| (A) | Polyoxyethylene (caprylate/caprate) glycerides (TEGOSOFT GMC 6, manufactured by Evonik Japan Co., Ltd.) | 3 | 0.3 | 10 | 1 | 5 |
| | Polyethylene glycol monolaurate (Emanon 1112HG, manufactured by Kao Corporation) | | | | | |
| (B) | Sodium N-stearoyl-N-methyltaurate (Kp = 50) (Nikkol SMT, manufactured by Nikko Chemicals Co., Ltd.) | | 0.1 | 1 | 0.03 | 0.1 |
| | Sodium laurate (Kp ≈ 38) | 0.3 | | | | |
| Solvent | Ethanol | 5 | 5 | 5 | 5 | 5 |
| (C) | Water | 91.7 | 94.6 | 84 | 93.97 | 89.9 |
| | Total | 100 | 100 | 100 | 100 | 100 |
| | (B)/(A) | 0.10 | 0.33 | 0.10 | 0.03 | 0.02 |

**EP 4 151 201 A1**

(continued)

| Component (mass%) | | Comparative Example | | | | |
|---|---|---|---|---|---|---|
| | | 1 | 2 | 3 | 4 | 5 |
| Effect | Dirt (makeup) removal | 4 | 2 | 5 | 3 | 4 |
| | Re-adhesion suppressing effect | 2 | 5 | 2 | 2 | 2 |
| | Skin after use (no stickiness) | 5 | 5 | 2 | 5 | 5 |
| | Skin after use (no residual feeling) | 5 | 5 | 5 | 5 | 5 |
| | Feeling of touching the skin | 4 | 4 | 4 | 4 | 4 |

[Table 4]

| Component (mass%) | | Example | | | |
|---|---|---|---|---|---|
| | | 20 | 21 | 22 | 23 |
| (A) | Polyoxyethylene (caprylate/caprate) glycerides (TEGOSOFT GMC 6, manufactured by Evonik Japan Co., Ltd.) | 3 | 3 | 3 | 3 |
| (B) | Sodium N-stearoyl-N-methyltaurate (Kp = 50) (Nikkol SMT, manufactured by Nikko Chemicals Co., Ltd.) | 0.3 | 0.3 | 0.3 | 0.3 |
| (D) | Acrylic acid-alkyl methacrylate copolymer Carbopol ETD2020, manufactured by Lubrizol Advanced Materials Inc.) | 0.1 | | 0.05 | 0.25 |
| | Hydroxypropyl cellulose (NISSO HPC, manufactured by Nippon Soda Co., Ltd.) | | 0.05 | | |
| Neutralizer | Potassium hydroxide | 0.02 | | 0.01 | 0.06 |
| (C) | Water | 96.58 | 96.65 | 96.64 | 96.39 |
| | Total | 100 | 100 | 100 | 100 |
| | (B)/(A) | 0.10 | 0.10 | 0.10 | 0.10 |
| Effect | Dirt (makeup) removal | 5 | 5 | 5 | 5 |
| | Re-adhesion suppressing effect | 5 | 5 | 5 | 5 |
| | Skin after use (no stickiness) | 5 | 5 | 5 | 5 |
| | Skin after use (no residual feeling) | 5 | 5 | 5 | 5 |
| | Feeling of touching the skin | 5 | 5 | 5 | 5 |

[Table 5]

| Non-woven sheet | |
|---|---|
| Hydrophobic fiber | Fiber obtained by heat fusion of PE using PET as core |
| Hydrophilic fiber | Cotton/rayon = 75/25 |
| Hydrophobic/hydrophilic ratio | 20/80 |
| Basis weight g/cm$^2$ | 45 |
| Thickness mm | 0.42 |
| Density g/cm$^3$ | 0.11 |
| Impregnation rate % | 450 |

Examples 24 to 29 and Comparative Examples 6 and 7

Non-woven fabric sheets shown in Table 6 were impregnated with the liquid composition of Example 4 to manufacture skin cleansing sheets as in Examples 1 to 23.

The resulting skin cleansing sheets were evaluated for the dirt (makeup) removal, re-adhesion suppressing effect, usability (feeling of touching the skin) as in Examples 1 to 23. In addition, liquid releasing was evaluated. The results are collectively shown in Table 6.

(Method for evaluating liquid releasing)

The entire area of an artificial skin (Bio Skin (trademark), manufactured by Beaulax Co., Ltd., dry type) having a size of 49 mm × 130 mm was wiped thoroughly (without overlapping in the wiping area) with a 4-fold skin cleansing sheet in one direction at a predetermined load (from 300 to 400 g). The amount of liquid adhered to the artificial skin was measured from a change in weight of the artificial skin between before and after the wiping. The liquid releasing was evaluated by the weight in accordance with the following criteria.

1: the amount of adhered liquid is less than 100 mg,
2: the amount of adhered liquid is 100 mg or more and less than 130 mg,
3: the amount of adhered liquid is 130 mg or more and less than 150 mg,
4: the amount of adhered liquid is 150 mg or more and less than 180 mg, and
5: the amount of adhered liquid is 180 mg or more.

[Table 6]

| | | Example | | | | | | Comparative Example | |
|---|---|---|---|---|---|---|---|---|---|
| Non-woven sheet | | 24 | 25 | 26 | 27 | 28 | 29 | 6 | 7 |
| Hydrophobic fiber (Fiber obtained by heat fusion of PE using PET as core) | | 100 | 90 | 70 | 30 | 20 | 10 | 0 | 0 |
| Hydrophilic fiber (cotton/rayon = 50/50) | | 0 | 10 | 30 | 70 | 80 | 90 | 100 | 100 |
| Impregnation rate | % | 450 | | | | | | | |
| Basis weight | g/m$^2$ | 45 | | | | | | | 60 |
| Thickness | mm | 0.6 | 0.57 | 0.53 | 0.44 | 0.42 | 0.4 | 0.37 | 0.4 |
| Density | g/cm$^3$ | 0.08 | 0.08 | 0.08 | 0.10 | 0.11 | 0.11 | 0.12 | 0.15 |
| Effect:Dirt (makeup) removal | | 5 | 5 | 5 | 5 | 4 | 4 | 3 | 3 |
| Re-adhesion suppressing effect | | 5 | 5 | 5 | 5 | 5 | 4 | 3 | 3 |
| Feeling of touching the skin | | 3 | 3 | 4 | 4 | 4 | 4 | 4 | 2 |
| Liquid releasing | | 5 | 5 | 5 | 4 | 4 | 4 | 3 | 2 |

Formulation Example 1

A non-woven fabric sheet shown in Table 5 was impregnated with a liquid composition having the composition shown in Table 7 to manufacture a skin cleansing sheet as in Examples 1 to 23.
The resulting skin cleansing sheet can suppress re-adhesion of dirt while floating dirt of the skin, such as makeup, from the skin, exhibit high detergency, and suppress the stickiness and residual feeling of the skin after use, and the feeling of touching the skin is also good.

[Table 7]

| Component (mass%) | | | Formulation Example |
|---|---|---|---|
| | | | 1 |
| (A) | | Polyoxyethylene (caprylate/caprate) glycerides (TEGOSOFT GMC 6, manufactured by Evonik Japan Co., Ltd.) | 3 |
| (B) | | Sodium N-stearoyl-N-methyltaurate (Kp = 50) (Nikkol SMT, manufactured by Nikko Chemicals Co., Ltd.) | 0.3 |
| Solvent | | Ethanol | 5 |
| Oil | | Jojoba oil | 8 |
| (C) | | Water | 83.7 |
| | | Total | 100 |
| | | (B)/(A) | 0.10 |

**Claims**

1. A sheet product comprising:
   a sheet, wherein the sheet comprises 5 mass% or more of a hydrophobic fiber and is impregnated with a liquid composition comprising components (A), (B), and (C) below:

   (A) a nonionic surfactant: from 0.5 to 9 mass%;
   (B) an anionic surfactant having a Krafft point of 45°C or more: from 0.05 to 3 mass% in terms of a salt; and
   (C) water: from 70 to 99.45 mass%, and wherein
   a ratio of a mass of the component (B) in terms of a salt to a mass of the component (A), (B)/(A), is from 0.03 to 1.0.

2. The sheet product according to Claim 1, wherein a content of the component (B) in the liquid composition is from 0.1 to 2.7 mass% in terms of a salt.

3. The sheet product according to Claim 1 or 2, wherein the ratio of the mass of the component (B) in terms of a salt to the mass of the component (A), (B)/(A), in the liquid composition is from 0.04 to 0.6.

4. The sheet product according to any one of Claims 1 to 3, wherein the liquid composition further comprises (D) a water-soluble polymer.

5. The sheet product according to Claim 4, wherein the water-soluble polymer as the component (D) includes an alkyl group having 3 or more carbon atoms.

6. The sheet product according to Claim 4 or 5, wherein a content of the component (D) in the liquid composition is from 0.01 to 0.3 mass%.

7. The sheet product according to any one of Claims 1 to 6, wherein the sheet comprises a hydrophilic fiber.

8. The sheet product according to any one of Claims 1 to 7, wherein the sheet comprises the hydrophilic fiber in an amount of 10 mass% or more.

9. The sheet product according to any one of Claims 1 to 8, wherein the sheet has a density of $0.14 \text{ g/cm}^3$ or less.

10. The sheet product according to any one of Claims 1 to 9, wherein the liquid composition further comprises (E) a powder.

11. The sheet product according to any one of Claims 1 to 10, wherein the sheet comprises a non-woven fabric.

12. The sheet product according to any one of Claims 1 to 11, wherein the sheet product is for skin cleansing.

13. The sheet product according to any one of Claims 1 to 12, wherein the sheet product is for removing makeup cosmetics.

| | INTERNATIONAL SEARCH REPORT | International application No. |
|---|---|---|
| | | PCT/JP2021/018505 |

**A. CLASSIFICATION OF SUBJECT MATTER**
A61K 8/39(2006.01)i; A61K 8/19(2006.01)i; A61K 8/25(2006.01)i; A61K
8/27(2006.01)i; A61K 8/29(2006.01)i; A61K 8/36(2006.01)i; A61K
8/46(2006.01)i; A61K 8/73(2006.01)i; A61K 8/81(2006.01)i; A61Q
1/14(2006.01)i; A61Q 19/10(2006.01)i
FI:　　A61K8/39; A61K8/19; A61K8/25; A61K8/27; A61K8/29; A61K8/36;
　　　A61K8/46; A61K8/73; A61K8/81; A61Q1/14; A61Q19/10
According to International Patent Classification (IPC) or to both national classification and IPC

**B. FIELDS SEARCHED**

Minimum documentation searched (classification system followed by classification symbols)
A61K8/00-8/99; A61Q1/00-90/00

Documentation searched other than minimum documentation to the extent that such documents are included in the fields searched
| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2005-343943 A (SHISEIDO CO., LTD.) 15 December 2005 (2005-12-15) paragraphs [0050]-[0056] | 1-13<br>1-13 |
| X<br>Y | WO 2015/186582 A1 (MANDOM CORPORATION) 10 December 2015 (2015-12-10) claims, paragraphs [0037]-[0073] | 1-6, 9-13<br>7-13 |
| Y | WO 2015/156387 A1 (KAO CORP.) 15 October 2015 (2015-10-15) paragraphs [0040]-[0084] | 1-13 |
| Y | JP 2001-314342 A (LION CORP.) 13 November 2001 (2001-11-13) paragraphs [0044]-[0045] | 1-13 |

☐ Further documents are listed in the continuation of Box C.    ☒ See patent family annex.

| | |
|---|---|
| *   Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| 07 July 2021 (07.07.2021) | 20 July 2021 (20.07.2021) |

| Name and mailing address of the ISA/<br>　Japan Patent Office<br>　3-4-3, Kasumigaseki, Chiyoda-ku,<br>　Tokyo 100-8915, Japan | Authorized officer<br><br>Telephone No. |
|---|---|

Form PCT/ISA/210 (second sheet) (January 2015)

**INTERNATIONAL SEARCH REPORT**
Information on patent family members

| International application No. |
| --- |
| PCT/JP2021/018505 |

| Patent Documents referred in the Report | Publication Date | Patent Family | Publication Date |
| --- | --- | --- | --- |
| JP 2005-343943 A | 15 Dec. 2005 | (Family: none) | |
| WO 2015/186582 A1 | 10 Dec. 2015 | KR 10-2016-0103139 A CN 106068118 A | |
| WO 2015/156387 A1 | 15 Oct. 2015 | CN 106163494 A | |
| JP 2001-314342 A | 13 Nov. 2001 | (Family: none) | |

Form PCT/ISA/210 (patent family annex) (January 2015)

**EP 4 151 201 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001314342 A